# EUROPEAN PATENT APPLICATION

(11) **EP 1 720 014 A1**
(43) Date of publication of application: **08.11.2006**
(21) Application number: 05719479.7
(22) Date of filing: 24.02.2005
(51) Int. Cl.: G01N 33/53, C12Q 1/02, G01N 33/15, G01N 33/50, C12N 15/09

(54) **METHOD OF JUDGING THE EXTENT OF VASCULOPATHY**

(30) Priority: 25.02.2004 JP 2004049444
(71) Applicant: Perseus Proteomics Inc., Tokyo 153-0041 (JP)
(72) Inventor: KODAMA, Tatsuhiko, c/o The University of Tokyo, Tokyo 153-8904 (JP); HAMAKUBO, Takao, c/o The University of Tokyo, Tokyo 153-8904 (JP); ITO, Yukio, c/o Perseus Proteomics Inc., Tokyo 1530041 (JP); IWANARI, Hiroko, c/o Perseus Proteomics Inc., Tokyo 153-0041 (JP); OHKUCHI, Masao, c/o Perseus Proteomics Inc., Tokyo 153-0041 (JP); SUGIYAMA, Akira, c/o Perseus Proteomics Inc., Tokyo 153-0041 (JP)
(74) Representative: Hartz, Nikolai
(86) International application number: PCT/JP2005/003029
(87) International publication number: WO 2005/080981

(57) **Abstract**

The invention provides a method of assessing a grade of vascular injury and a method of screening a medicament for vascular injury. The method of assessing a grade of vascular injury is **characterized by** including determination of PTX3 level in a test sample.

Sequence Listing

<110>  Perseus Proteomics Inc.

<120>  Method for determining degree of angiopathy

<130>  PER0002

<150>  JP 2004-049444
<151>  2004-02-25

<160>  2

<170>  PatentIn version 3.1

<210>  1
<211>  1145
<212>  DNA
<213>  Homo sapiens

<400>  1
atgcatctcc ttgcgattct gttttgtgct ctctggtctg cagtgttggc cgagaactcg      60

gatgattatg atctcatgta tgtgaatttg gacaacgaaa tagacaatgg actccatccc     120

actgaggacc ccacgccgtg cgactgcggt caggagcact cggaatggga caagctcttc     180

atcatgctgg agaactcgca gatgagagag cgcatgctgc tgcaagccac ggacgacgtc     240

ctgcggggcg agctgcagag gctgcgggag gagctgggcc ggctcgcgga aagcctggcg     300

aggccgtgcg cgccgggggc tcccgcagag gccaggctga ccagtgctct ggacgagctg     360

ctgcaggcga cccgcgacgc gggccgcagg ctggcgcgta tggagggcgc ggaggcgcag     420

cgcccagagg aggcggggcg cgccctggcc gcggtgctag aggagctgcg gcagacgcga     480

gccgacctgc acgcggtgca gggctgggct gcccggagct ggctgccggc aggttgtgaa     540

acagctattt tattcccaat gcgttccaag aagatttttg gaagcgtgca tccagtgaga     600

ccaatgaggc ttgagtcttt tagtgcctgc atttgggtca aagccacaga tgtattaaac     660

aaaaccatcc tgttttccta tggcacaaag aggaatccat atgaaatcca gctgtatctc     720

agctaccaat ccatagtgtt tgtggtgggt ggagaggaga acaaactggt tgctgaagcc     780

atggtttccc tgggaaggtg gacccacctg tgcggcacct ggaattcaga ggaagggctc     840

acatccttgt gggtaaatgg tgaactggcg gctaccactg ttgagatggc cacaggtcac     900

attgttcctg agggaggatc ctgcagattg gccaagaaaa gaatggctgc tgtgtgggtg     960

gtggctttga tgaaacatta gccttctctg ggagactcac aggcttcaat atctgggata    1020

gtgttcttag caatgaagag ataagagaga ccggaggagc agagtcttgt cacatccggg    1080

ggaatattgt tgggtgggga gtcacagaga tccagccaca tggaggagct cagtatgttt    1140

cataa                                                                1145

<210>  2
<211>  381

<212>  PRT
<213>  Homo sapiens

<400>  2

Met His Leu Leu Ala Ile Leu Phe Cys Ala Leu Trp Ser Ala Val Leu
1               5                   10                  15

Ala Glu Asn Ser Asp Asp Tyr Asp Leu Met Tyr Val Asn Leu Asp Asn
            20                  25                  30

Glu Ile Asp Asn Gly Leu His Pro Thr Glu Asp Pro Thr Pro Cys Asp
        35                  40                  45

Cys Gly Gln Glu His Ser Glu Trp Asp Lys Leu Phe Ile Met Leu Glu
    50                  55                  60

Asn Ser Gln Met Arg Glu Arg Met Leu Leu Gln Ala Thr Asp Asp Val
65                  70                  75                  80

Leu Arg Gly Glu Leu Gln Arg Leu Arg Glu Glu Leu Gly Arg Leu Ala
                85                  90                  95

Glu Ser Leu Ala Arg Pro Cys Ala Pro Gly Ala Pro Ala Glu Ala Arg
            100                 105                 110

Leu Thr Ser Ala Leu Asp Glu Leu Leu Gln Ala Thr Arg Asp Ala Gly
        115                 120                 125

Arg Arg Leu Ala Arg Met Glu Gly Ala Glu Ala Gln Arg Pro Glu Glu
    130                 135                 140

Ala Gly Arg Ala Leu Ala Ala Val Leu Glu Glu Leu Arg Gln Thr Arg
145                 150                 155                 160

Ala Asp Leu His Ala Val Gln Gly Trp Ala Ala Arg Ser Trp Leu Pro
                165                 170                 175

Ala Gly Cys Glu Thr Ala Ile Leu Phe Pro Met Arg Ser Lys Lys Ile
            180                 185                 190

Phe Gly Ser Val His Pro Val Arg Pro Met Arg Leu Glu Ser Phe Ser
        195                 200                 205

Ala Cys Ile Trp Val Lys Ala Thr Asp Val Leu Asn Lys Thr Ile Leu
    210                 215                 220

Phe Ser Tyr Gly Thr Lys Arg Asn Pro Tyr Glu Ile Gln Leu Tyr Leu
225                 230                 235                 240

Ser Tyr Gln Ser Ile Val Phe Val Val Gly Gly Glu Glu Asn Lys Leu
                245                 250                 255

Val Ala Glu Ala Met Val Ser Leu Gly Arg Trp Thr His Leu Cys Gly
            260                 265                 270

Thr Trp Asn Ser Glu Glu Gly Leu Thr Ser Leu Trp Val Asn Gly Glu
        275                 280                 285

Leu Ala Ala Thr Thr Val Glu Met Ala Thr Gly His Ile Val Pro Glu
    290                 295                 300

Gly Gly Ile Leu Gln Ile Gly Gln Glu Lys Asn Gly Cys Cys Val Gly
305                 310                 315                 320

Gly Gly Phe Asp Glu Thr Leu Ala Phe Ser Gly Arg Leu Thr Gly Phe
                325                 330                 335

Asn Ile Trp Asp Ser Val Leu Ser Asn Glu Glu Ile Arg Glu Thr Gly
            340                 345                 350

Gly Ala Glu Ser Cys His Ile Arg Gly Asn Ile Val Gly Trp Gly Val
        355                 360                 365

Thr Glu Ile Gln Pro His Gly Gly Ala Gln Tyr Val Ser
    370                 375                 380

<210>  3
<211>  30
<212>  DNA
<213>  Artificial Sequence
<220>
<223>  Primer sequence for amplification of NM_002852

<400> 3
cggggtatgc atctccttgc gattctgttt                                    30

<210>  4
<211>  38
<212>  DNA
<213>  Artificial Sequence
<220>
<223>  Primer sequence for amplification of NM_002852

<400>  4
cgcggatcct tatgaaacat actgagctcc tccatgtg                           38

Sequence Listing

<110>  Perseus Proteomics Inc.

<120>  Method for determining degree of angiopathy

<130>  PER0002

<150>  JP 2004-049444
<151>  2004-02-25

<160>  2

<170>  PatentIn version 3.1

<210>  1
<211>  1145
<212>  DNA
<213>  Homo sapiens

<400>  1
atgcatctcc ttgcgattct gttttgtgct ctctggtctg cagtgttggc cgagaactcg      60

gatgattatg atctcatgta tgtgaatttg gacaacgaaa tagacaatgg actccatccc     120

actgaggacc ccacgccgtg cgactgcggt caggagcact cggaatggga caagctcttc     180

atcatgctgg agaactcgca gatgagagag cgcatgctgc tgcaagccac ggacgacgtc     240

ctgcggggcg agctgcagag gctgcgggag gagctgggcc ggctcgcgga aagcctggcg     300

aggccgtgcg cgccgggggc tcccgcagag gccaggctga ccagtgctct ggacgagctg     360

ctgcaggcga cccgcgacgc gggccgcagg ctggcgcgta tggagggcgc ggaggcgcag     420

cgcccagagg aggcggggcg cgccctggcc gcggtgctag aggagctgcg gcagacgcga     480

gccgacctgc acgcggtgca gggctgggct gcccggagct ggctgccggc aggttgtgaa     540

acagctattt tattcccaat gcgttccaag aagatttttg gaagcgtgca tccagtgaga     600

ccaatgaggc ttgagtcttt tagtgcctgc atttgggtca aagccacaga tgtattaaac     660

aaaaccatcc tgttttccta tggcacaaag aggaatccat atgaaatcca gctgtatctc     720

agctaccaat ccatagtgtt tgtggtgggt ggagaggaga acaaactggt tgctgaagcc     780

atggtttccc tgggaaggtg gacccacctg tgcggcacct ggaattcaga ggaagggctc     840

acatccttgt gggtaaatgg tgaactggcg gctaccactg ttgagatggc cacaggtcac 900

attgttcctg agggaggatc ctgcagattg gccaagaaaa gaatggctgc tgtgtgggtg 960

gtggctttga tgaaacatta gccttctctg ggagactcac aggcttcaat atctgggata 1020

gtgttcttag caatgaagag ataagagaga ccggaggagc agagtcttgt cacatccggg 1080

ggaatattgt tgggtgggga gtcacagaga tccagccaca tggaggagct cagtatgttt 1140

cataa 1145

<210> 2
<211> 381
<212> PRT
<213> Homo sapiens

<400> 2

Met His Leu Leu Ala Ile Leu Phe Cys Ala Leu Trp Ser Ala Val Leu
1               5                   10                  15

Ala Glu Asn Ser Asp Asp Tyr Asp Leu Met Tyr Val Asn Leu Asp Asn
            20                  25                  30

Glu Ile Asp Asn Gly Leu His Pro Thr Glu Asp Pro Thr Pro Cys Asp
        35                  40                  45

Cys Gly Gln Glu His Ser Glu Trp Asp Lys Leu Phe Ile Met Leu Glu
    50                  55                  60

Asn Ser Gln Met Arg Glu Arg Met Leu Leu Gln Ala Thr Asp Asp Val
65                  70                  75                  80

Leu Arg Gly Glu Leu Gln Arg Leu Arg Glu Glu Leu Gly Arg Leu Ala
                85                  90                  95

Glu Ser Leu Ala Arg Pro Cys Ala Pro Gly Ala Pro Ala Glu Ala Arg
            100                 105                 110

Leu Thr Ser Ala Leu Asp Glu Leu Leu Gln Ala Thr Arg Asp Ala Gly

115                 120                 125

Arg Arg Leu Ala Arg Met Glu Gly Ala Glu Ala Gln Arg Pro Glu Glu
    130                 135                 140

Ala Gly Arg Ala Leu Ala Ala Val Leu Glu Glu Leu Arg Gln Thr Arg
145                 150                 155                 160

Ala Asp Leu His Ala Val Gln Gly Trp Ala Ala Arg Ser Trp Leu Pro
                165                 170                 175

Ala Gly Cys Glu Thr Ala Ile Leu Phe Pro Met Arg Ser Lys Lys Ile
            180                 185                 190

Phe Gly Ser Val His Pro Val Arg Pro Met Arg Leu Glu Ser Phe Ser
        195                 200                 205

Ala Cys Ile Trp Val Lys Ala Thr Asp Val Leu Asn Lys Thr Ile Leu
    210                 215                 220

Phe Ser Tyr Gly Thr Lys Arg Asn Pro Tyr Glu Ile Gln Leu Tyr Leu
225                 230                 235                 240

Ser Tyr Gln Ser Ile Val Phe Val Val Gly Gly Glu Glu Asn Lys Leu
                245                 250                 255

Val Ala Glu Ala Met Val Ser Leu Gly Arg Trp Thr His Leu Cys Gly
            260                 265                 270

Thr Trp Asn Ser Glu Glu Gly Leu Thr Ser Leu Trp Val Asn Gly Glu
        275                 280                 285

Leu Ala Ala Thr Thr Val Glu Met Ala Thr Gly His Ile Val Pro Glu
    290                 295                 300

Gly Gly Ile Leu Gln Ile Gly Gln Glu Lys Asn Gly Cys Cys Val Gly
305                 310                 315                 320

Gly Gly Phe Asp Glu Thr Leu Ala Phe Ser Gly Arg Leu Thr Gly Phe
                325                 330                 335

Asn Ile Trp Asp Ser Val Leu Ser Asn Glu Glu Ile Arg Glu Thr Gly
            340                 345                 350

Gly Ala Glu Ser Cys His Ile Arg Gly Asn Ile Val Gly Trp Gly Val
        355                 360                 365

Thr Glu Ile Gln Pro His Gly Gly Ala Gln Tyr Val Ser
    370                 375                 380

<210>  3
<211>  30
<212>  DNA
<213>  Artificial Sequence
<220>
<223>  Primer sequence for amplification of NM_002852

<400> 3
cggggtatgc atctccttgc gattctgttt                                    30

<210>  4
<211>  38
<212>  DNA
<213>  Artificial Sequence
<220>
<223>  Primer sequence for amplification of NM_002852

<400>  4
cgcggatcct tatgaaacat actgagctcc tccatgtg                           38

## Description

### [Background Art]

The present invention relates to a method of assessing a grade of vascular injury and to a method of screening a medicament for vascular injury.

### [Technical Field]

PTX3, which is sometimes called "Pentraxin", "Pentaxin", "TSG-14" or "MPTX3", is a secreted protein that belongs to the pentraxin family and is known to develop in human umbilical cord vessel endothelial cells stimulated with interleukin 1 (IL-1) (Non-Patent Document 1).

The pentraxin family includes C-reactive protein (CRP) and serum amyloid P component (SAP), and both are known as an inflammation-related protein. Pentraxin is also known as "long pentraxin", whereas CRP is also known as "short Pentraxin". Thus, pentraxin forms a family which shares a conserved domain, CRP, and this family is thought to presumably function of inflammation. CRP or SAP is induced by IL-6, whereas PTX3 is not susceptible to induction by IL-6. Furthermore, PTX3 is expressed in different types of cells with those compared with the expression patter of CRP or SAP. This difference may explain PTX3 has a different function with that of CRP and SAP (Non-Patent Document 2).

Meanwhile, it has been discovered that PTX3 sharply increases in the blood of a patient suffering from an acute myocardial infarction, known as one of inflammation reactions, and PTX3 was possible indicator for small vasculitis). Moreover, PTX3 was commonly detectable in an advanced arteriosclerosis plaques with immunostaining suggesting that PTX3 is implicated in the onset of inflammation (Non-Patent Document 3).

The term "inflammation" refers to a wide range of inflammation including dermatitis and those of a variety of organs. Of these, vasculitis could lead to a severe disease such as heart disease or cerebral disease. In respect of heart diseases, the risk factors for an acute myocardial infarction include high level in total cholesterol, hypertension, diabetes, obesity, and smoking. In order to remove such risk factors, for example, a patient at a high level of total cholesterol is usually treated not only by a diet therapy, but also by use of statin known as a hyperlipidemia suppressant. Cerebral disease include dementia induced by, vascular injury and such disease is treated with aspirin.

As one of the conventional methods for detecting PTX3, the ELISA method disclosed in Non-Patent Document 3 is well known. This document describes that PTX3 level reaches its maximum (22 ng/mL) at 7.5 hours after onset of myocardial infarction, and then drastically decreases to 0.5 to 2.5 ng/mL, which is a level exhibited by healthy people probably not suffering heart disease. As hence evidenced from the results obtained by the conventional method, it can be confirmed that the blood PTX3 level increases at least during a myocardial infarction, but it remains to be clarified how the level of PTX3 changes at an earlier stage than the onset of myocardial infarction and dementia in relation to cerebral vascular injury.

There have already been some medicaments known for vascular injury, such as statins (medicaments for hyperlipidemia) and aspirin (an analgesic antipyretic agent). However, while these medicaments were confirmed to serve for the improvement of vascular injury by an epidemiological survey, no drug targeted primarily for the cure of vascular injury has been developed so far.
Non-Patent Document 1: Breviorio et al.: J. Biol. Chem., 267(31), 22190-7(1992)
Non-Patent Document 2: J. Biol. Chem., 267(31), 22190-7(1992); Domyaku Koka (Arteriosclerosis), 24(7-8), 375-80(1996)
Non-Patent Document 3: Arthritis and Rheumatism, 44/12 (2841-50), 2001, Circulation, 102, 636-41(2000), Arterioscler Thromb Vasc Biol. 2002; 22: e10-e14

### [Disclosure of the Invention]

### [Problems to be Solved by the Invention]

If the severity of vascular injury can be detected before the onset of myocardial infarction or dementia in relation to cerebrovascular disease, it is possible to determine the severity of heart disease or cerebrovascular disease and thereby evaluate effects of a medicament on these diseases. In addition, if any method is possible to assess progressive grade of vascular injury, this method becomes a good screening system to evaluate medicament for vascular injury.
Thus, an object of the present invention is to provide a method for assesing the grade of vascular injury, which is a risk factor for myocardial infarction or dementia in relation to cerebrovascular injury. Another object of the invention is to provide a method of screening a medicament for vascular injury.

### Means for Solving the problems

Under the above circumstances, the present inventors focused on measuring blood PTX3 level, which is only increased at an acute stage of myocardial infarction, and went on to measure the blood PTX3 protein level before and after administration of statin or aspirin, which are the agents to medicate vascular injury. As a result, the PTX3 level turned out to decrease after administration of the above drugs. The inventors also determined plasma PTX3 levels in patients under myocardial infarction or other possible symptoms therefor (i.e., stable angina and unstable angina). As a result, the PTX3 level turned out to highly increase in parallel with the gravity of vascular injury.
Meanwhile, in view of the acknowledged fact that CRP, which is an inflammation-related protein and belongs to the pentraxin family including PTX3, is usable for the diagnosis of the common cold, the present inventors investigated blood PTX3 levels of patients suffering common cold. As a result, no significant increase in the blood PTX3 level was observed, as opposed to that of CRP. From this fact, the inventors found that PTX3 was not a diagnosis marker for systemic inflammatory reaction, but proposed as a beneficial diagnosic marker exhibiting high vessel-specificity and indicating the extent of vascular injury. Furthermore, the inventors also found that the above indication could be applied for a method of screening a medicament to cure vascular injury. Thus the present invention was accomplished on the basis of these findings.

In the studies of the present inventors, a novel anti-PTX3 antibody was produced to establish a high-sensitive assay system, and serum PTX3 level was determined before and after administration of statin or aspirin. Significant decrease in PTX3 level was confirmed after administration. Thus the present invention was accomplised.

Accordingly, the present invention provides a method of assesing the grade of vascular injury, characterized by comprising determination of PTX3 level in a test sample.
The present invention also provides a diagnostic agent for assessing the grade of vascular injury, the agent comprising an anti-PTX3 antibody.
The present invention also provides a method of screening a medicament for vascular injury, characterized in that the method comprises administering a test sample to an animal or bringing a test sample into contact with a cell, and determining variation in amount of PTX3 protein or a PTX3 gene.

### [Effects of the Invention]

According to the present invention, the extent of vascular injury can be diagnosed before the onset of acute myocardial infarction, vascular injury-related dementia, or a similar disease, whereby severity of heart disease or cerebovascular disease can be judged, and therefore, progression to severe heart disease or cerebral disease can be prevented. In addition, a treatment regimen for vascular injury employing a drug can be established. Also, a new medicament for heart or cerebral vascular injury can be screened.

### Brief Description of the Drawings

[Fig. 1] Comparison between recombinant PTX3 and natural PTX3 by Western blot analysis.
[Fig. 2] A graph showing newly provided ELISA standard curves.
[Fig. 3] A graph showing comparison between a newly provided PTX3 assay system and a conventional PTX3 assay system, when applied to assay of clinical samples.
[Fig. 4] A graph showing blood PTX3 levels of heart disease patients.

### [Best Modes for Carrying Out the Invention]

As used herein, the term "determination" refers to both quantitative and non-quantitative measurement. Examples of non-quantitative measurement include measurement to simply check presence of PTX3 protein, measurement to determine presence of PTX3 protein in a predetermined amount or more, and measurement to determine the amount of PTX3 protein as compared with other samples (e.g., a control sample). Examples of the quantitative measurement include determination of PTX3 protein level and determination of the amount of PTX3 protein. The nucleotide sequence of PTX3 gene is shown in SEQ ID NO: 1, and the amino acid sequence of PTX3 protein is shown in SEQ ID NO: 2.

No particular limitation is imposed on the type of the assay sample, so long as the sample may contain PTX3 protein. Preferably, the sample is collected from an living body such as a mammal, more preferably from a human. Specific examples of the assay sample include blood, interstitial fluid, plasma, extravascular fluid, cerebrospinal fluid, synovial fluid, pleural fluid, serum, lymph, saliva, and urine. Of these, blood, serum, and plasma are preferred. Further, assay samples such as culture medium of cells collected from a living body also fall within the scope of the analysing sample of the present invention.

Vascular injury progresses to cause a severe disease, via injury of vascular endothelial cells, migration of smooth muscle cells from tunica media, recruitment of macrophages and formation of formy cells, adhesion of thrombi, plaque formation, fibrosis of blood vessels, and rupture of plaques.
In the present invention, vascular injury includes vascular injury caused by hyperlipidemia, cerebral disease, hypertension, diabetes, obesity, and smoking.
In the present invention, the extent of vascular injury refers to the extent of disorders involved in the above-specified progression of vascular injury. In other words, the extent of vascular injury progressing in the above course until rupture of plaques is rated by the following pathological histological parameters: (a) lipid core size, (b) thickness of fibrous cap, (c) strength of shear stress, and (d) the extent of inflammatory infiltration. Plaques are more readily ruptured, as (a) increases, (b) is thinner, (c) increases, and (d) increases. Thus, the extent of vascular injury in the present invention is rated by the extent of the aforementioned (a) to (d).

In the method of the present invention, PTX3 is preferably determined through immunological assay employing an anti-PTX3 antibody. The determination employing an anti-PTX3 antibody will next be described in detail.

### 1. Production of an anti-PTX3 antibody

No particular limitation is imposed on the origin, type (monoclonal, polyclonal), and morphology of the anti-PTX3 antibody employed in the present invention, so long as it is specifically bound to PTX3 protein. Specifically, known antibodies such as a mouse antibody, a rat antibody, a human antibody, a chimera antibody, and a humanized antibody may be employed. Although these antibodies may be polyclonal, monoclonal antibodies are preferred.
The anti-PTX3 antibody immobilized on a carrier and the anti-PTX3 antibody labeled with a labeling substance may recognize the same epitope of the PTX3 molecule. Preferably, these antibodies recognize different epitopes.

The anti-PTX3 antibody employed in the present invention may be obtained by known techniques to establish a polyclonal or monoclonal antibody. The anti-PTX3 antibody employed in the present invention is particularly preferably a mammalian monoclonal antibody. The mammalian monoclonal antibody includes that produced from a hybridoma and that produced by a host genetically transformed with an expression vector containing an antibody gene.

Generally, the hybridoma producing a monoclonal antibody may be produced by a known technique in the following procedure. Specifically, PTX3 is employed as an immunogen, and the antigen is immunized through a known method. The thus-obtained immunocytes are fused with known parent cells by a conventional cell fusion method. After a customary screening method, cells producing a monoclonal antibody can be established.

Specifically, monoclonal antibodies may be produced in the following procedure.
Firstly, PTX3 employed as an immunogen for producing the antibody is obtained by purification of a supernatant of a culture of available cells. Alternatively, PTX3 may also be obtained through a method disclosed in Japanese Kohyo Patent Publication No. 2002-503642.
The thus-purified PTX3 protein is employed as an immunogen. Alternatively, a partial peptide of the PTX3 may also be employed as an immunogen. In the latter case, the partial peptide may be produced through chemical synthesis from an amino acid sequence of human PTX3; may be produced from an expression vector including a part of the PTX3 gene; or may be produced through degradation of naturally occurring PTX3 by use of a proteolytic enzyme. No particular limitation is imposed on the part and size of the partial peptide of PTX3.

No particular limitation is imposed on the mammal to be immunized with an immunogen, and the mammal is preferably selected in consideration of adaptability to parent cells employed in cell fusion. Generally, monkeys, and rodents such as mice, rats, hamsters, and rabbits are employed.

Immunization of an animal with an immunogen is performed through a known method. For example, in a generally employed method, the immunogen is administered to a mammal through intraperitoneal or subcutaneous injection. More specifically, an immunogen is diluted with and suspended in an appropriate amount of PBS (Phosphate-Buffered Saline), physiological saline, or a similar medium, and if required, an appropriate amount of a conventional adjuvant (e.g., Freund's complete adjuvant) is added to the suspension. The mixture is emulsified and administered to the mammal several times per day every 4 to 21 days. Upon immunization with an immunogen, an appropriate carrier may also be used. Particularly when a partial peptide having a small molecular weight is employed as an immunogen, the partial peptide is preferably bound to a carrier protein such as albumin or keyhole lympet hemocyanin for immunization.

The mammal is immunized in the above manner. After confirmation that the serum antibody level has been elevated to a desired level, immunocytes are collected from the mammal, and the collected cells are subjected to cell fusion. Among immunocytes, spleen cells are particularly preferred.

The parent cells to be fused with the aforementioned immunocytes are myeloma cells of a mammal. The myeloma cells to be preferably employed are known cell strains such as P3(P3x63Ag8.653) (J. Immnol. (1979) 123, 1548-1550), P3x63Ag8U.1 (Current Topics in Microbiology and Immunology (1978)81, 1-7), NS-1 (Kohler. G. and Milstein, C. Eur. J. Immunol. (1976) 6, 511-519), MPC-11 (Margulies. D. H. et al., Cell (1976) 8, 405-415), SP2/0 (Shulman, M. et al., Nature (1978)276, 269-270), FO (de St. Groth, S. F. et al., J. Immunol. Methods (1980) 35, 1-21), S194 (Trowbridge, I. S. J. Exp. Med. (1978)148, 313-323), and R210(Galfre, G. et al., Nature (1979) 277, 131-133).

Basically, cell fusion between the aforementioned immunocytes and the myeloma cells may be performed through a known method; for example, a method of Kohler. G. and Milstein, C., disclosed in Methods Enzymol.(1981) 73, 3-46.

More specifically, the cell fusion is performed in a conventional nutritive culture medium in the presence of a cell fusion accelerator. Examples of the fusion accelerator to be employed include polyethylene glycol (PEG) and Sendai virus (HVJ). If desired, an auxliary substance such as dimethyl sulfoxide may be added to enhance fusion efficiency.

The ratio of immunocytes to myeloma cells may be predetermined arbitrarily. For example, immunocytes are preferably used in an amount 1 to 10 times that of myeloma cells. Examples of the culture medium applicable in the cell fusion such as RPMI1640 or MEM, which is suitable for proliferation of myeloma cell strains, and other conventional culture medium employed in such cell culturing. In addition, serum supplements such as fetal calf serum (FCS) may be used in combination.

In the cell fusion, predetermined amounts of the immunocytes and the myeloma cells are sufficiently mixed together in the culture medium, and a 30-60%(w/v) solution of polyethylene glycol (PEG) (e.g., average molecular weight: about 1,000 to 6,000), which has been heated to about 37°C in advance, is added to the cell mixture, whereby target hybridomas are formed. Subsequently, an appropriate culture medium is successively added, and the supernatant is complemently removed by serial centrifugation, to thereby remove a cell fusion reagent or other substances unsuited for the growth of the hybridomas.

The thus-obtained hybridomas are selected in selective culture with medium (e.g., HAT medium (medium containing hypoxanthine, aminopterin, and thymidine)). The culture in the HAT medium is continued for a period of time sufficient to kill cells other than target hybridomas (non-fused cells), generally for several days to several weeks. Subsequent screening and mono-cloning, hybridomas producing the target antibody is performed by a conventional limiting dilution method.

Screening and mono-cloning of the target antibody may be performed through a known screening method based on antigen-antibody reaction. For example, an antigen is immobilized on a carrier such as polystyrene beads or a 96-well microtiter plate, and a culture supernatant of the hybridomas is added to the plate to react with the antigen. After washing the carrier, an enzyme-labeled second antibody or a similar antibody is added to determine whether an antibody reacting with the immunogen is contained in the culture supernatant. The hybridomas producing the target antibody can be cloned through the limiting dilution method or a similar method. In this case, an antigen employed for immunization may be used.
Instead of the method for producing the hybridoma through immunization of an animal other than human with an antigen, a target human antibody exhibiting binding activity to PTX3 may be obtained through another method (see Japanese patent Publication (kokoku) No. 1-59878). In the method, human lymphocytes are sensitized in vitro with PTX3, and sensitized lymphocytes are fused with human myeloma cells, which has permanent cell division potential. Still alternatively, PTX3 (antigen) is administered to a transgenic animal having all the repertories of human antibody genes, whereby cells producing the anti-PTX3 antibody are collected. The cells are immortalized, and a human antibody with respect to PTX3 is obtained (see International Patent Application Laid-Open Nos. WO94/25585, WO93/12227, WO92/03918, and WO94/02602).

The thus-produced hybridomas producing a monoclonal antibody can be subcultured in a conventional culture medium, and stored in liquid nitrogen for a long period of time.

When a monoclonal antibody is obtained from the hybridomas, the hybridomas are cultured in a conventional method, and the culture supernatant is collected. Alternatively, the hybridomas are administered to a mammal adaptable to the hybridomas and proliferated in the body, and resultant ascites are collected. The former method is suitable for producing a high-purity monoclonal antibody, whereas the latter method is suitable for mass production of a monoclonal antibody.

In the present invention, there may be employed a recombinant monoclonal antibody which has been produced by a recombination technique, including cloning an antibody gene from hybridomas, integrating the gene into an appropriate vector, and introducing the vector into a host cell(see, for example, Vandamme, A. M. et al., Eur. J. Biochem. (1990) 192, 767-775, 1990) .
Specifically, mRNA coding for a variable (V) region of an anti-PTX3 antibody is isolated from the hybridomas producing the anti-PTX3 antibody. Isolation of mRNA may be performed through a known method. For example, the total RNA is prepared by a method such as the guanidine ultracentrifugation method (Chirgwin, J. M. et al., Biochemistry (1979) 18, 5294-5299) or the AGPC method (Chomczynski, P. et al., Anal. Biochem. (1987) 162, 156-159), and mRNA of interest is prepared by means of an mRNA Purification Kit (product of Pharmacia) or a similar kit. Alternatively, mRNA can be directly prepared by use of a QuickPrep mRNA Purification Kit (product of Pharmacia).

From the thus-produced mRNA, cDNA of the antibody V region is synthesized by use of reverse transcriptase. Synthesis of cDNA is performed by use of a kit such as AMV Reverse Transcriptase First-strand cDNA Synthesis Kit (product of Seikagaku Kogyo). Synthesis and amplification of cDNA may be performed by use of 5'-Ampli FINDER RACE Kit (product of Clontech) and a PCR method; specifically, a 5'-RACE method (Frohman, M. A. et al., Proc. Natl. Acad. Sci. USA (1988) 85, 8998-9002, Belyavsky, A. et al., Nucleic Acids Res. (1989) 17, 2919-2932).

A target DNA fragment is purified from the thus-obtained PCR product, and the fragment is ligated to the vector DNA. Recombinant vectors are produced therefrom, and those of interest are prepared through introducing the recombinant vectors into *E. Coli.* or other hosts and selecting colonies. The target nucleotide sequence of the DNA is confirmed through a known method; for example, the dideoxynucleotide chain termination method.

After production of a DNA coding for the V region of the target anti-PTX3 antibody, the DNA is introduced into an expression vector containing a DNA coding for a desired antibody constant region (C region).

In the production of the anti-PTX3 antibody employed in the present invention, an antibody gene is introduced into an expression vector such that the antibody is expressed in an expression-regulated region under control conditions (e.g., by an enhancer or a promoter). Then, the host cells are transformed by the expression vector, to thereby express the antibody.

Expression of the antibody gene may be performed through incorporating a first DNA coding for an antibody heavy chain (H chain) and a second DNA coding for an antibody light chain (L chain) separately into expression vectors, whereby the host cells are transformed simultaneously. Alternatively, a DNA coding for both the H chain and the L chain is introduced into a single expression vector, whereby the host cells are transformed (see WO94/11523).

In addition to the aforementioned host cells, a transgenic animal may be employed to produce a recombinant antibody. For example, an antibody gene is inserted in a gene coding for a protein intrinsically produced in milk (e.g., goat β-casein), to thereby prepare a fused gene. A DNA fragment including the fused gene in which the antibody gene has been inserted is injected into a goat embryo, and the embryo is introduced into a female goat. As a result, a transgenic goat born from the goat which has accepted the embryo or its progeny thereof produces milk from which the target antibody is obtained. In order to increase the amount of target-antibody-containing milk produced by the transgenic goat, a hormone may be appropriately administered to the transgenic goat (Ebert, K. M. et al., Bio/Technology (1994) 12, 699-702).

In the present invention, in addition to the aforementioned antibody, an artificially modified recombinant antibody (e.g., a chimeric antibody or a humanized antibody) may also be used. These modified antibodies may be produced through a known method.

The chimeric antibody is produced by ligating the above-obtained DNA coding for an antibody V region to a DNA coding for a human antibody C region, incorporating the DNA into an expression vector, and introducing the vector into a host cell. Through the conventional process, the chimeric antibody useful for the present invention can be produced.

A humanized antibody, which is also called a reshaped human antibody, is obtained through transferring a CDR (complementarity determining region) of a non-human mammal (e.g., mouse) antibody into a CDR of a human antibody. General recombination techniques relating to the above process are known (see European Patent Application Laid-Open No. EP 125023 and WO96/02576).

Specifically, a DNA sequence which has been constructed so as to bind a CDR of a mouse antibody and a framework region (FR) of a human antibody is synthesized through PCR employing several oligonucleotides serving as primers, each oligonucleotide having been produced such that it has a portion overlapping the terminal region of the DNA coding for CDR and the terminal region of DNA coding for FR (see the methodology disclosed in WO98/13388).

The FR of a human antibody to be ligated via a CDR is selected such that CDR form a favorable antigen binding site. In accordance with needs, an amino acid residue in the FR in the antibody variable region may be substituted such that a CDR of the reshaped human antibody forms an appropriate antigen binding site (Sato, K. et al., Cancer Res. (1993) 53, 851-856).

The C region on a chimera antibody or a humanized antibody is derived from a human antibody. For example, an H chain may employ Cγ1, Cγ2, Cγ3, or Cγ4, and an L chain may employ Cκ or Cλ. In order to improve the stability of an antibody or its production process thereof, the human antibody C region may be modified.

A chimeric antibody consists of a variable region of a non-human mammal antibody and a constant region of a human antibody. A humanized antibody consists of a CDR of a non-human mammal antibody and a FR and a C region of a human antibody. Since the humanized antibody has a reduced antigenicity in the human body, it is useful for an active component of the medicament of the present invention.

The antibody employed in the present invention may be a whole molecule of the antibody. Alternatively, the antibody may be a fragment or a modified product of the antibody, so long as PTX3 is bound thereto. The antibody also includes a divalent antibody and a monovalent antibody. Examples of antibody fragments include Fab, F(ab')2, Fv, Fab/c containing one Fab and the complete Fc, and single-chain Fv (scFv) in which Fv of the H chain or that of the L chain are linked via an appropriate linker. Specifically, an antibody is treated with an enzyme such as papain or pepsin, to thereby form antibody fragments. Alternatively, a gene coding for these antibody fragments is constructed; the gene is introduced to an expression vector; and the vector is expressed in appropriate host cells (see, for example, Co, M. S. et al., J. Immunol. (1994) 152, 2968-2976, Better, M. & Horwitz, A. H. Methods in Enzymology (1989) 178, 476-496, Academic Press,Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. et al., Methods in Enzymology (1989) 121, 663-669, Bird, R. E. et al., TIBTECH (1991)9, 132-137).

Fragment scFv is obtained by linking the V region of the antibody H chain and the V region of the L chain. In scFv, the V region of the H chain and the V region of the L chain are linked to each other via a linker, preferably a peptide linker (Huston, J. S. et al., Proc. Natl. Acad. Sci. U.S.A. (1988) 85, 5879-5883). In scFv, the V region of the H chain and the V region of the L chain may be derived from any of the antibodies described in the present specification. As a peptide linker linking the V regions, any single strand peptide having, for example 12 to 19 amino acid residues, is employed.

The DNA coding for scFv may be produced through the following procedure. Specifically, from a DNA coding for the H chain of the aforementioned antibody or the V region of the H chain, and a DNA coding for the L chain or the V region of the L chain, a DNA fragment coding for the total or a desired part of the amino acid sequence is selected as a template. The template is amplified through PCR by use of a primer pair defining the two ends. Subsequently, amplification is continued by use of a DNA coding for the peptide linker portion and a primer pair defining such that the ends of the linker are linked with the H chain and the L chain.

Once the DNA coding for scFv has been produced, conventional methods can be employed to produce an expression vector containing the DNA, as well as a host transformed by the expression vector. Through use of the host, scFv can be produced through a standard method.

Similarly, these antibody fragments can be produced through obtaining a gene, expression of the gene, and production by a host. In the present invention, the term "antibody" also includes a fragment of the antibody.

As a modified product of an antibody, an anti-PTX3 antibody, which is bound to one of various molecules such as a labeling substance, may also be employed. In the present invention, the term "antibody" also includes such antibody modified products. These antibody modified products may be obtained through chemical modification of the obtained antibody. Notably, the method for modifying antibodies has been well established in the art.

The antibody employed in the present invention may be a bispecific antibody. The bispecific antibody may have antigen-binding sites which recognize different epitopes on a PTX3 molecule. Alternatively, one antigen-binding site recognizes PTX3, and the other antigen-binding site recognizes a substance such as a labeling substance. The bispecific antibody may be produced through binding two antibody HL pairs, or through fusing hybridomas producing different monoclonal antibodies, thereby producing fusion cells producing a bispecific antibody. Alternatively, the bispecific antibody may be produced through a genetic engineering technique.

Expression of the thus-produced antibody gene may be performed through a known method, whereby the corresponding antibody can be produced. In the case of mammal cells, the antibodies can be expressed by operably linking a useful conventional promoter, the antibody gene to be expressed, and a poly A signal at the 3' downstream thereof. Examples of the promoter/enhancer include human cytomegalovirus immediate early promoter/enhancer.

Examples of other promoter/enhancers for expressing the antibody employed in the present invention include virus promoter/enhancers such as retrovirus, polyoma virus, adenovirus, simian virus40 (SV40); and promoter/enhancers derived from mammal cells such as human elongation factor 1a (HEF1a).

When SV40 promoter/enhancer is employed, gene expression can be readily attained through the method of Mulligan et al. (Nature (1979) 277, 108), whereas when HEF1a promoter/enhancer is employed, gene expression can be readily attained through the method of Mizushima et al. (Nucleic Acids Res. (1990) 18, 5322).

In the case of *E*. *coli,* A useful conventional promoter, a signal sequence for antibody secretion and an antibody gene are operably ligated, so that the gene can be expressed. Examples of the promoter include lacZ promoter and araB promoter. When lacZ promoter is employed, expression can be performed through the method of Ward et al. (Nature (1098) 341, 544-546; FASEB J. (1992) 6, 2422-2427), whereas when araB promoter is employed, expression can be performed through the method of Better et al. (Science (1988) 240, 1041-1043).

As a signal sequence for secreting an antibody, pelB signal sequence (Lei, S. P. et al J. Bacteriol. (1987) 169, 4379) may be used, in the case where the antibody is produced in periplasm of *E. coli.* Subsequently, the antibody produced in periplasm is isolated, and the structure of the antibody is appropriately refolded for use.

As a replication origin, those derived from SV40, polyoma virus, adenovirus, bovin pailloma virus (BPV), etc. may be employed. In order to amplify the copy number of the gene in a host cell system, the expression vector may contain, as a selection marker, an aminoglycoside transferase (APH) gene, a thymidine kinase (TK) gene, an *E. coli* xanthine guanine phosphoribosyl transferase (Ecogpt) gene, or a dihydrofolic acid reductase (dhfr) gene.

For the production of the antibody employed in the present invention, any expression systems; e.g., a eucaryotic cell system or a procaryotic cell system, may be employed. Examples of the eucaryotic cell include established cells such as a mammalian cell system, an insect cell system, a mycotic cell system, and a yeast cell system, and examples of the procaryotic cell include bacterial cell systems such as an *E*. *coli* cell system.

Preferably, the antibody employed in the present invention is expressed in mammal cells such as CHO cells, COS cells, myeloma cells, BHK cells, Vero cells, and HeLa cells.
Subsequently, transformed host cells are cultured in vitro or in vivo, whereby a target antibody is produced. Culturing host cell is performed through a known method. Examples of the culture media employed include DMEM, MEM, RPMI 1640, and IMDM. A serum supplement such as fetal calf serum (FCS) may be used in combination.

The resultant antibody is isolated from a cell or a host animal, and may be purified to a homogeneous condition. Isolation and purification of the antibody employed in the present invention may be performed by means of an affinity column. Examples of protein A columns include Hyper D, POROS, and Sepharose F.F. (product of Pharmacia). However, no particular limitation is imposed on the isolation/purification method, so long as it is generally employed for isolating and purifying protein. Besides the aforementioned affinity column, the antibody may be isolated and purified through an appropriate combination of a chromatography column, a filter, ultrafiltration, salting out, dialysis, etc. (Antibodies A Laboratory Manual. Ed Harlow, David Lane, Cold Spring Harbor Laboratory, 1988).

### 2. Determination of PTX3

No particular limitation is imposed on the PTX3 to be determined in the present invention, and full-length PTX3 and a fragment thereof may be determined.
No particular limitation is imposed on the method of detecting PTX3 protein contained in an assay sample. However, PTX3 is preferably detected through immunoassay by use of an anti-PTX3 antibody. Examples of immunoassay include radioimmunoassay, enzyme immunoassay, fluoroimmunoassay, luminescence immunoassay, and immunoprecipitation, immuno-nephelometry, Western blotting, immunostaining, and immunodiffusion techniques. Of these, enzyme immunoassay is preferred, with enzyme-linked immunosorbent assay (ELISA) (e.g., sandwich ELISA) being particularly preferred. The aforementioned immunoassay methods including ELISA may be carried out through a technique known in the art.

In one general detection method employing an anti-PTX3 antibody, an anti-PTX3 antibody is immobilized on a carrier, and an assay sample is added to the carrier. Through incubation, PTX3 protein is bound to the anti-PTX3 antibody. After washing the carrier, PTX3 protein bound the anti-PTX3 antibody on the carrier is detected, whereby PTX3 protein level in the assay sample can be quantitated.

Examples of the carrier employed in the present invention include carriers made of insoluble polysaccharide such as agarose or cellulose; synthetic resin such as silicone resin, polystyrene resin, polyacrylamide resin, nylon resin, or polycarbonate resin; or insoluble material such as glass. In use, these carriers may be in the form of beads or a plate. In the case of beads, a column filled with the beads may be employed. In the case of plate, a multi-well plate (e.g., 96-well multi-well plate), a biosensor chip, etc., may be employed. The anti-PTX3 antibody may be bound to a carrier through a conventional method such as chemical binding or physical adsorption. Needless to say, commercial products of these carriers may be employed.

Generally, binding of PTX3 protein to the anti-PTX3 antibody is performed in a buffer. Examples of the buffer employed include phosphate buffer, Tris buffer, citrate buffer, borate buffer, and carbonate buffer. Incubation is performed under generally employed conditions; for example, 4°C to room temperature for one hour to 24 hours. After incubation, the carrier is washed. No particular limitation is imposed on the washing material, and any material may be used, so long as it does not block binding of PTX3 protein to the anti-PTX3 antibody. For example, a buffer containing a surfactant such as Tween20 is employed.

In the method of determining PTX3 protein according to the present invention, a control sample may be placed in addition to an assay sample for detection of PTX3 protein. Examples of the control sample include a negative control sample containing no PTX3 protein and a positive control sample containing PTX3 protein. In the case where control samples are employed, analytical results obtained by the negative control sample containing no PTX3 protein and those obtained by the positive control sample containing PTX3 protein are compared, whereby PTX3 protein present in the assay sample can be detected. In an alternative method, a series of control samples having stepwise-graded concentrations are prepared. Through analysis of these control samples, a standard curve is obtained from the detection data. With reference to the standard curve, PTX3 protein contained in an assay sample can be quantified from the absolute values.

In one preferred embodiment, PTX3 protein bound to the carrier via an anti-PTX3 antibody is determined by use of an anti-PTX3 antibody labeled with a labeling substance.

For example, an assay sample is applied for exposing to the anti-PTX3 antibody immobilized on the carrier. After washing the carrier, PTX3 protein is detected by a labeled antibody which specifically recognizes PTX3 protein.

Labeling of the anti-PTX3 antibody may be performed through a generally known method. Labeling substances such as a fluorescent dye, an enzyme, a co-enzyme, a chemoluminescent substance, a radioactive substance, etc., which are known in the art as labeling substances, may be employed. Specific examples include radioisotopes (e.g., ³²P, ¹⁴C, ¹²⁵I, ³H, and ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lyzozyme, saccharide oxidase, microperoxidase, and biotin. When biotin is used as a labeling substance, enzyme (e.g., alkaline phosphatase)-bound avidin is preferably added after addition of biotin-labeled antibody. Binding of the labeling substance to the anti-PTX3 antibody may be performed through a known method such as the glutaraldehyde method, the maleimide method, the pyridyldisulfide method, or the periodate method.

In a specific procedure, a solution containing an anti-PTX3 antibody is added to a carrier, such as a plate, thereby immobilizing the anti-PTX3 antibody on the carrier. After washing of the plate, the antibody is blocked with BSA, gelatin, albumin, etc. in order not to bind non-specific protein to the antibody. The plate is washed again, then an assay sample is added to the plate. After incubation, the plate is washed, and another labeled anti-PTX3 second antibody is added. After incubation for an appropriate time, the plate is washed again, and the labeled anti-PTX3 antibody specifically bound mediated by PTX3 protein on the plate is detected. Detection may be performed through a method known in the art. For example, in the case in which the labeling substance is a radioactive substance, the antibody can be detected through liquid scintillation or the RIA method.
When the labeling substance is an enzyme, a substrate is added, and enzymatic change in the substrate, indicated by, for example, coloring, can be detected by a spectrophotometer. Examples of the substrate include 2,2-azinobis(3-ethylbenzothiazoline-6-sulfonate) diammonium salt (ABTS), 1,2-phenylenediamine(o-phenylenediamine), and 3,3',5,5'-tetramethylbenzidine (TME). When the labeling substance is a fluorescent substance, the antibody can be detected by means of a fluorometer.

One preferred embodiment of the method of determining PTX3 protein of the present invention employs a biotin-labeled anti-PTX3 antibody and avidin.

In a specific procedure, a solution containing an anti-PTX3 antibody is added to a carrier such as a plate, thereby immobilizing the anti-PTX3 antibody on the carrier. After washing of the plate, the antibody is blocked by BSA or a similar substance in order not to bind nonspecific protein to the antibody coated carrier. The plate is washed again, and an assay sample is added to the plate. After incubation, the plate is washed, and a biotin-labeled anti-PTX3 antibody is added. After incubation for an appropriate time, the plate is washed again, and avidin conjugated to an enzyme such as alkaline phosphatase or peroxidase is added. After incubation, the plate is washed, and a substrate corresponding to the enzyme conjugated to avidin is added. PTX3 protein is detected by an index such as enzymatic change in the substrate.

Another embodiment of the method of determining PTX3 protein of the present invention is a method in which at least one primary antibody specifically recognizes PTX3 protein, and at least one secondary antibody specifically recognizes the primary antibody.

In a specific procedure, an assay sample is subject to at least one anti-PTX3 antibody immobilized on the carrier. After incubation, the carrier is washed. PTX3 protein remaining after washing is detected by a primary anti-antibody and at least one secondary antibody specifically recognizes PTX3 captured on the primary antibody. In this case, the secondary antibody is preferably labeled with a labeling substance.

Still another embodiment of the method of determining PTX3 protein of the present invention is a detection method employing agglutination reaction. In the method, PTX3 can be detected by a carrier sensitized with an anti-PTX3 antibody. No particular limitation is imposed on the type of the carrier which sensitizes the antibody, and any carrier may be employed, so long as the carrier is insoluble, causes no non-specific reaction, and is stable. For example, latex particles, bentonite, collodion, kaolin, fixed sheep erythrocytes, etc. may be employed. Of these, latex particles are preferably employed. Examples of employable latex particles include polystyrene latex particles, styrenebutadiene copolymer latex particles, and polyvinyltoluene latex particles. Of these, polystyrene latex particles are preferably employed. Sensitized particles and a sample are mixed together, and the mixture is stirred for a predetermined period of time. The higher the concentration of PTX3 contained in the sample is, the larger the agglutination degree of the particles is obserbed. Thus, PTX3 can be detected through visual observation under the size of the agglutination. Alternatively, turbidity increased as the carrier forms larger the agglutination,which is measured by a spectrophotometer or a similar device, to thereby detect PTX3.

Yet another embodiment of the method of determining PTX3 protein of the present invention is a method employing a biosensor on the basis of surface plasmon resonance. The biosensor employing surface plasmon resonance enables real-time observation of protein-protein interaction as a surface plasmon resonance signal by use of a small amount of protein without labeling. For example, through employment of a biosensor such as BIA core (product of Pharmacia), binding between PTX3 protein and an anti-PTX3 antibody can be detected. Specifically, an assay sample is brought into contact with a sensor chip on which an anti-PTX3 antibody has been immobilized, whereby PTX3 protein bound to the anti-PTX3 antibody can be detected as change in a resonance signal.

The detection method of the present invention may be automated by use of a variety of automated inspection apparatuses. Thus, a large number of samples may be assayed at once.

An object of the present invention is to provide a diagnostic drug for judging the extent of vascular injury. The diagnostic agent essentially contains an anti-PTX3 antibody. As used herein, the term "diagnostic agent" also encompasses a kit. When the diagnostic agent is used on the basis of the ELISA method, the agent may contain a carrier for immobilizing an antibody. Alternatively, an antibody may be bound to the carrier in advance. When the diagnostic agent is used on the basis of the agglutination method employing a carrier such as latex, the agent may contain an antibody-conjugated carrier thereon. In addition, the diagnostic agent may further contain a blocking solution, reaction solution, reaction-terminating solution, or reagents for treating samples, in accordance with needs.

The method of screening a medicament for vascular injury includes administering an assay sample to an animal, or bringing an assay sample into contact with a cell, and determining the PTX3 protein level or the PTX3 gene level. When an animal is employed, an assay sample is administered orally, through intravenous injection, or through other means. The blood PTX3 protein level or the blood PTX3 gene level is determined before and after administration of assay sample, and the results are compared with those of the non-administered group, whereby the medicament can be screened. In this case, model animals for hyperlipidemia, cerebral disease, diabetes, obesity, and hypertension may be employed. Among them, particularly preferably employed model animals are high fat diet-loaded animals, WHHL rabbits, apo-E defect mice, LDL receptor defect mice, human apo-B-introduced mice, dominant mutation apo-E-introduced mice, high blood LDL pigs, and miniature pigs, etc. (arteriosclerosis models); and SHRSP rats (cerebral disease models); leptin defect mice, NOD mice, KK/Ta Jcl mice, KK-Ay/Ta Jcl mice, C57BL/KsJ-db/db Jcl mice, C57BL/KsJ-db/+m Jcl mice, and GK/Jcl rats (obesity/diabetes models); SHR rats (hypertension onset model); and vascular injury rats or mice (for example coronary artery balloon damage model etc.) (heart disease models).

In the case where a cell is employed, a PTX3 producing cell line and cells forcedly expressing PTX3 are cultured with an assay sample, and the thus-produced PTX3 protein or a PTX3 gene is determined through a known method. The results are compared with a sample-free case, whereby the medicament can be screened.

PTX3 protein may be determined through a known method such as the ELISA method. Examples of the genetic determination method include quantitative RT-PCR. In order to screen a substance for suppressing PTX3 gene expression, generally employed reporter gene assay may also be employed. In the generally employed method, a vector in which a promoter region of the PTX3 gene is ligated to the upstream region of cDNA of a marker protein such as luciferase, galactosidase, or GFP is produced, and the vector is transfected to animal cells through a conventional method. An assay sample is added to the transfected animal cells for culturing, and the marker protein is detected through an appropriate method depending on the type of marker, whereby a target compound can be screened.

### [Examples]

The present invention will next be described in detail by way of Examples, which should not be construed as limiting the invention thereto.

### Example 1 Cloning of PTX3

Cloning of a sequence including a full-length ORF region of PTX3 was performed. Single-strand cDNA of a human umbilical cord vessel endothelial cell (HUVEC) was prepared through the aforementioned method (described in [0034]). Through the PCR employing primers PTX3-F (KpnI) (SEQ ID NO: 3) and PTX3-R (BamHI) (SEQ ID NO: 4), which had been constructed from GenBank No. NM_002852, and the above single-strand cDNA serving as a template, a full-length ORF gene was isolated. The fragment obtained through the PCR was inserted to a vector by means of Zero Blunt TOPO PCR Cloning Kit, and nucleotide sequence was analyzed through a routine method. The vector was cleaved at KpnI site and BamHI site, to thereby obtain a fragment, and the fragment was inserted into a phCMV vector (product of Stratagene), whereby a transfer vector phCMV-PTX3 was produced.

### Example 2 Construction of cells expressing recombinant PTX3

According to the protocol FuGENE 6 (by Roche Molecular Biochemicals), CHO cells (1×10⁵ cells) were seeded on a 6-well dish on the day before transfection, and the cells were cultured overnight. Next day, the expression vector phCMV-PTX3 (8 µg) and FuGENE 6 reagent (16 µL) were added to a serum-free DMEM medium (100 µL), followed by incubation at room temperature for 20 minutes. The culture was added to the above cells. On the day after transfection, cloning was performed through the limiting dilution method employing G418 serving as a selection reagent. Culture supernatant of each clone was collected, and PTX3 protein expressing cells were screened. As a result, a clone stably expressing PTX3 protein in an amount of about 2 to 3 µg/mL was selected. Hereinafter, the stable clone is represented by CHO-PTX3.

### Example 3 Production of recombinant PTX3 protein

Purification of protein was performed in accordance with the method of Bottazzi *et al.* (Bottazzi B, Vouret-Craviari, et al., J Biol Chem. 1997; 272(52): 32817-23.). Specifically, CHO-PTX3 was cultured in a 150-cm² flask and, subsequently, culture was further performed for four days in roller bottles (product of BD Bioscience), each holding a serum-free medium (S-SFM-II, product of GIBCO/Invitrogen) (300 mL), while rotating speed was controlled to 1 rpm. The culture supernatants were collected, and the combined culture supernatant (1 L) was concentrated to 50 mL by means of an ultrafiltration membrane apparatus, Pellicon XL device Biomax 100 (product of MILLIPORE). The concentrate was dialyzed twice against 50mM imidazole/buffer (pH 6.6) (5 L). Subsequently, the dialyzed concentrate was subjected to ionexchange chromatography employing HiPrep 16/10 QXL (product of Pharmacia Biotech, Uppsala, Sweden). The column was washed until the background level was satisfactorily lowered. The NaCl concentration was increased from 0 to 0.58M over 35 minutes, and PTX3 was eluted with 1M NaCl. The eluate was monitored by absorbance at 280 nm. Fractions containing PTX3 protein were combined, the combined fraction was subjected to gel filtration chromatography employing Sephacryl S-300,and the column was developed with PBS. In order to selectively collect polymer of PTX3 among various monomer or multimated forms of secreted PTX3 protein, Superose 6 column (product of Pharmacia Biotech) was used. Specifically, calibration was performed by use of a molecular weight standard, and PTX3 was applied to the column. Development was performed with PBS at a flow rate of 0.4 mL/min. Eluate was analyzed through SDS-PAGE, whereby purified recombinant polymer of PTX3 protein was obtained.

### Example 4 Production of natural PTX3 protein

PTX3 protein was obtained from a supernatant of HUVEC culture. Specifically, HUVECs were cultured at 37°C for three days in an EGM-2 medium containing 5% FBS placed in a 175-cm² flask, and the culture medium was collected. Purification of protein was performed according to the method of Bottazzi *et al*. (Bottazzi B, Vouret-Craviari, et al., J Biol Chem. 1997; 272 (52): 32817-23.). The specific procedure was the same as that employed in Example 3. Thus, natural PTX3 was obtained.

### Example 5 Comparison between recombinant PTX3 and natural PTX3 through Western blotting

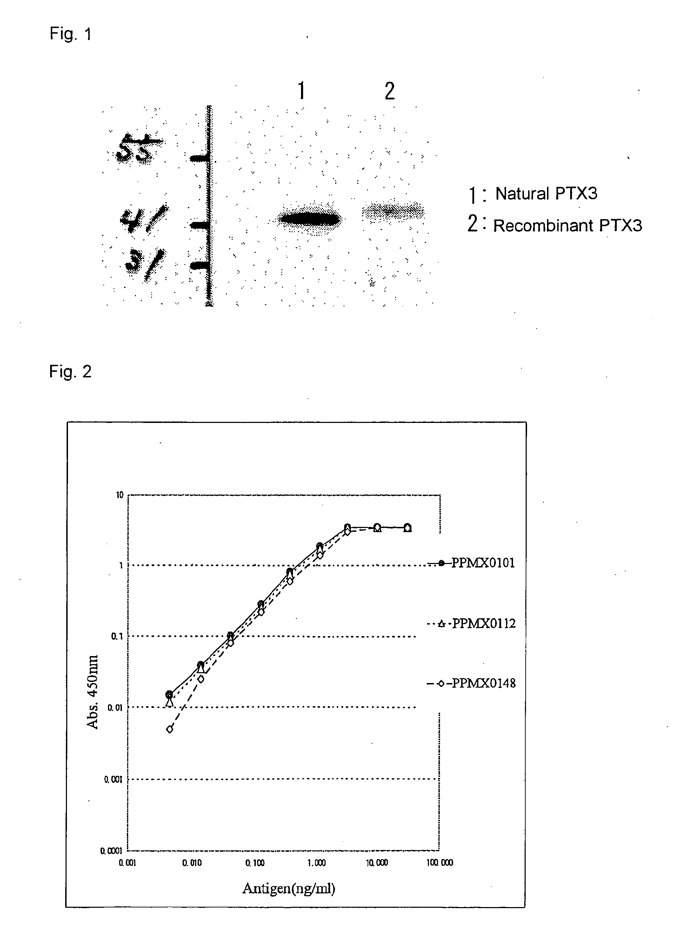

The above-obtained natural PTX3 was compared with the above-produced recombinant PTX3 through Western blotting. In a specific procedure, a 0.5-µg/mL natural PTX3 solution (10 µL) and a 0.5-µg/mL recombinant PTX3 solution (10 µL) were subjected to SDS-PAGE under reducing conditions. After completion of electrophoresis, the gel was transferred onto a PVDF membrane. PTX3 transferred onto the membrane was reacted with an anti-PTX3 polyclonal antibody (product of ALEXIS). Subsequently, the anti-PTX3 polyclonal antibody was reacted with an HRP-labeled anti-rabbit IgG antibody, whereby bands attributed to PTX3 were detected. Fig. 1 shows the results. As shown in Fig. 1, two bands are almost coincided with each other in terms of molecular weight. Slight variation may be attributed to the difference in sugar chain.

### Example 6 Production of anti-PTX3 monoclonal antibody

The monoclonal antibody was produced through the following procedure. Specifically, Balb/C mice (CRL) or PTX3 knockout mice were immunized with PTX3. In priming, a protein as immunogen was prepared so that the dose was adjusted to 10 or 100 µg/mouse, and emulsified with FCA (Freund's complete adjuvant,H37 Ra),(Difco:3113-60 or Becton Dickinson:cat# 231131). The emulsion was subcutaneously injected. Two weeks after priming, a protein as immunogen was prepared so that the dose was adjusted to 5 or 50 µg/mouse, and emulsified with FIA (Freund's incomplete adjuvant), (Difco:0639-60 or Becton Dickinson:cat# 263910). The emulsion was subcutaneously injected. Thereafter, as a booster, the emulsion was administered twice with an interval of one week. In final immunization, the protein was diluted with PBS so that the dose was adjusted to 5 or 50 µg/mouse, followed by administration to the tail vein. Through the ELISA employing a PTX3-protein-coated immunoplate, saturation of the titer (in serum) of the anti-PTX3 antibody was confirmed. Subsequently, mouse myeloma cells P3U1 and spleen cells from immunized mouse were fused by useing PEG 1500 (product of Roche Diagnostics, cat# 783641). The fused cells were seeded on a 96-well culture plate containing an HAT medium and, were selectively cultured from the next day. After completion of selective culture, the supernatant was screened through the ELISA. Positive clones to the ELISA were isolated monoclonally through limiting dilution and cultured extensively, whereby the culture supernatant was collected. Screening through the ELISA was performed on the basis of an index; i.e., binding activity between an antibody and PTX3 protein. Thus, a number of anti-PTX3 antibodies exhibiting high binding activity were obtained.

The monoclonal antibodies were purified by use of Hi Trap ProteinG HP (product of Amersham, CAT# 17-0404-01). Specifically, a supernatant of hybridoma medium was directly applied to the above column and washed with a binding buffer (20mM sodium phosphate (pH 7.0)), followed with an eluting buffer (0.1M glycin-HCl (pH 2.7)). The eluate was collected to a tube where a neutralizing buffer (1M Tris-HCl (pH 9.0)) had been added for immediate neutralization. Antibody-eluted fractions were pooled and dialyzed against 0.05% Tween20/PBS for one day (for buffer substitution). In the thus-purified antibodies, NaN₃ were added so as to adjust to 0.02%, and stored at 4°C.

Isotyping of anti-PTX3 monoclonal antibodies was carried out through the following procedure.
The antibody content was determined through mouse IgG sandwich ELISA employing a goat anti-mouse IgG (gamma) (produce of ZYMED, CAT# 62-6600) and alkaline phosphatase-a goat anti-mouse IgG (gamma) (ZYMED CAT# 62-6622), with a commercial purified mouse IgG1 antibody (product of ZYMED CAT# 02-6100) as a standard. Isotyping of anti-PTX3 antibodies was performed by ImmunoPure Monoclonal Antibody Isotyping, Kit II (product of PIERCE, CAT# 37502) in accordance with the attached manual in the kit. Through isotyping antibodies, each class categorized as IgG1, IgG2a, or IgM was identified.

### Example 7 Production of anti-PTX3 polyclonal antibody

New Zealand white rabbits (10-weeks old, body weight: 2 kg) were immunized. In priming, a protein as immunogen was prepared so that the PTX3 protein dose was adjusted to 100 µg/ rabbit, and emulsified with FCA (Freund's complete adjuvant,H37 Ra),(Difco:3113-60 or Becton Dickinson:cat# 231131). The emulsion was subcutaneously injected. Two weeks after priming, a protein as immunogen was prepared so that the dose was adjusted to 100 µg/ rabbit, and emulsified with FIA (Freund's incomplete adjuvant), (Difco:0639-60 or Becton Dickinson:cat# 263910). Thereafter, FIA emulsion (100 µmg/ rabbit) was administered five times to the rabbit at intervals of two weeks. After completion of the fifth booster, a part of blood was collected. The antibody titer (in rabbit serum) was evaluated through ELISA employing an antigen-coated 96-well plate. Subsequently, whole blood was collected, and serum was prepared from the blood. Through purification with Protein G column chromatography, fractions containing antibodies were obtained. In the thus-purified antibodies, NaN₃ were added to 0.02%, and stored at 4°C.

### Example 8 Construction of a ELISA system and a measurement employing the system

In order to detect PTX3 in blood, a sandwich ELISA system for PTX3 was constructed in the following manner. Specifically, a monoclonal antibody such as PPMX 0101 (FERM P-19697), PPMX 0102, PPMX 0112, or PPMX 0148 was coated on a 96-well plate, and a biotin-labeled polyclonal antibody (product of ALEXIS or antibody produced in Example 7) was employed as an antibody for detecting the PTX3 protein captured on the coated antibody. Biotinylation of the antibodies was performed by use of Sulfo-NHS-LC-Biotin (product of Pierce: CAT# 21335).

The anti-PTX3 antibody was diluted against PBS(-)and it was adjusted to a final concentration of 10 µg/mL, and the diluted antibody was coated on a 96-well immunoplate, followed by incubation at 4°C overnight. The next day, the plate was washed three times with a washing buffer (0.05% (v/v) Tween 20, PBS) (300 µL/well), and blocked with an immunoassay stabilizer (product of ABI, ABI #10-601-001) (150 µL). The coated plate was maintained for several hours at room temperature, or overnight at 4°C. Purified standard protein and testing sample such as human sera, were appropriately diluted with a diluting buffer (50mM Tris-HCl pH 8.0, 0.15M NaCl) containing animal serum or other components. The diluted samples were added to the plate, followed by incubation at room temperature for two hours. Subsequently, a biotin-labeled anti-PTX3 antibody which had been diluted to 20 µg/mL with PBS(-) containing an animal serum or other components was added to the plate, followed by incubation at room temperature for two hours. After removal of reactive residue, streptavidin-HRPO (product of Vector, Vector #SA-5004) which had been diluted to 3 µg/mL with Stab-ELISA-r (product of Cygnus, Cygnus #I-030) containing an appropriate amount of animal serum was added to the plate, followed by incubation at room temperature for 0.5 hours.
The plate was washed five times with a washing buffer (300 µL/well), and samples were colored by use of TMB (product of Scytek, Cat# TM4999) in accordance with the attached protocol. The absorbance was measured by means of a microplate reader.

The PTX3 protein level of each sample was calculated from the absorbance value through spreadsheet calculation software, GraphPad PRISM (product of GraphPad software Inc., ver. 3.0). A standard curve was drawn up from the analytical results of purified PTX3. As a result, a plurality of assay systems enabling to determine a lower PTX3 protein level was constructed (Fig. 2).

### Example 9 Comparison of a standard curve in new assay system with that in a conventional system

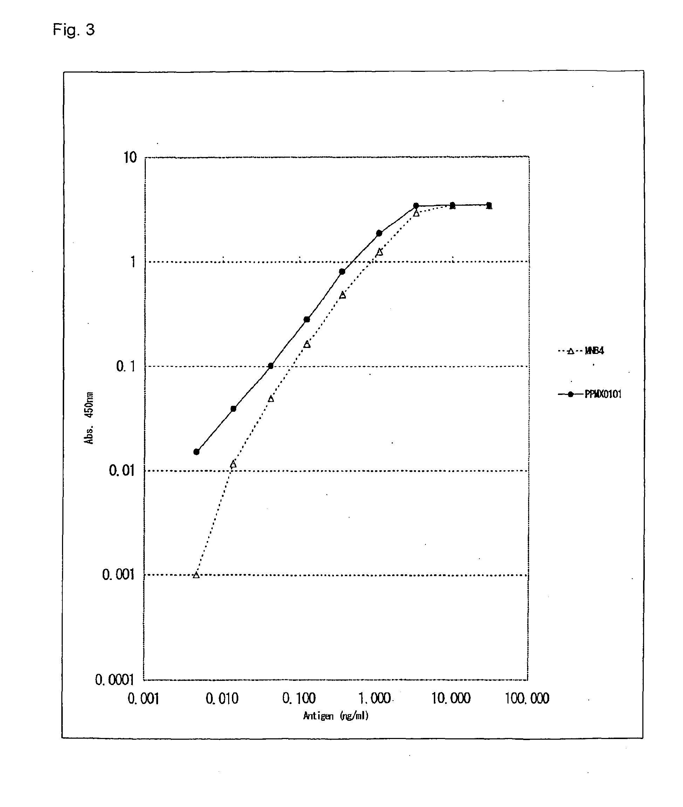

A standard curve employed in the ELISA was drawn up by use of serial dilute solution (concentrations: 30, 10, 3, 1, 0.3, 0.1, 0.03, 0.01 ng/mL) of a standard substance being concentration-verified PTX3 protein (product of ALEXIS). In order to compare sensitivity in the ELISA, a standard curve was drawn up in the case of an antibody disclosed in Non-Patent Document 3; i.e., MNB4 antibody (product of ALEXIS) in the above manner. Fig. 3 is a graph showing comparison between a PTX3 assay system established in Example 9 and an assay system employing the antibody disclosed in Non-Patent Document 3. As is clear from Fig. 3, the assay system established in Example 9 attains higher sensitivity.

### Example 10 Statin administration test

A blood sample was collected from each test specimen before administration of a statin. To each test specimen, atorvastatin was administered once per day after taking an evening meal for one week, and subsequently, a blood sample was collected. The thus-collected blood samples were incubated at 37°C for two hours, and centrifuged at 1,000 rpm for 10 minutes, to thereby obtain serum. The serum was collected and stored at -80°C. By use of these sera, the serum PTX3 protein level was determined with the ELISA system. All six assayed samples exhibited a decrease in serum PTX3 protein level (Table 1). Through the t-test, the PTX3 level one week after administration of atorvastatin was found to be significantly (p<0.01) reduced.

[Table 1]

**Table 1 Serum PTX3 level (ng/mL)**

| No. | Before administration | After administration |
|---|---|---|
| 1 | 0.31 | 0.26 |
| 2 | 0.40 | 0.23 |
| 3 | 0.35 | 0.20 |
| 4 | 0.36 | 0.14 |
| 5 | 0.44 | 0.12 |
| 6 | 0.42 | 0.10 |

### Example 11 Selection of blood samples

Conventionally, serum and plasmas (citric acid, EDTA, and heparin) were employed as blood samples for blood component assay. In the present invention, a serum sample, which was the most general blood collection method have been analyzed. In this experiment, a variety of blood PTX3 level depending on the type of blood collection format was investigated through an ELISA system employing a PPMX 0112 monoclonal antibody with addition of a PTX3 standard substance. The results are shown in Table 2. As is clear from Table 2, employment of EDTA was found to be the most appropriate blood collection format.

**[Table 2]**

| Amount of antigen | Absorbance 450 nm | | | |
|---|---|---|---|---|
| | Blood collection format | | | |
| | serum | heparin | EDTA | citric acid |
| 0 | 0.05 | 0.084 | 0.513 | 0.034 |
| 5 ng/mL | 0.032 | 0.056 | 1.387 | 0.061 |
| 10 ng/mL | 0.111 | 0.171 | 3.462 | 0.249 |

### Example 12 Stability test of blood samples

Most blood components are stable in the frozen state thereof. However, some substances may fail to maintain stability. Thus, stability of PTX3 in plasma at -80°C was investigated through an ELISA system employing a PPMX 0112 monoclonal antibody with adding known amount of PTX3 standard. The results are shown in Table 3. As is clear from Table 3, decrease in measured value was observed in the course of storage.

**[Table 3]**

| Panel specimen Added PTX3 level | Level (PTX3 ng/mL) |
|---|---|
| 0 ng/mL | 1.21 |
| 2 ng/mL | 2.32 |
| 5 ng/mL | 3.25 |
| 10 ng/mL | 5.23 |

### Example 13 Improvement in measuring conditions

The test carried out in Example 12 has revealed that storage stability of PTX3 at -80°C cannot be maintained. Possible reasons of unstableness in the storage are not only a degeneration of PTX3 but a change in other plasma components. In an attempt to suppress the latter, addition of EDTA or a protease inhibitor to a buffer used in measurement was investigated through an ELISA system employing a PPMX 0112 monoclonal antibody. As a result, addition of EDTA was found to improve PTX3 values measured (Table 4). In addition to EDTA, a surfactant such as Tween 20 or Bridje was further added to each plasma sample. As is clear from Table 5, addition of Tween 20 enhances the effect attained by addition of EDTA.
Based on the results, decrease in PTX3 content during storage may be attributed to a change of plasma components other than PTX3.

**[Table 4]**

| Panel specimen Added PTX3 level | Absence of EDTA (PTX3 ng/mL) | Presence of EDTA (PTX3 ng/mL) |
|---|---|---|
| 0 ng/mL | 1.21 | 1.21 |
| 2 ng/mL | 2.32 | 3.99 |
| 5 ng/mL | 3.25 | 5.93 |
| 10 ng/mL | 5.23 | 9.51 |

**[Table 5]**

| Panel specimen Added PTX3 level | Sole EDTA (PTX3 ng/mL) | EDTA+Tween20 (PTX3 ng/mL) |
|---|---|---|
| 0 ng/mL | 1.21 | 1.32 |
| 2 ng/mL | 3.99 | 4.62 |
| 5 ng/mL | 5.93 | 6.59 |
| 10 ng/mL | 9.51 | 10.27 |

### Example 14 Selection of a monoclonal antibody for use in ELISA

By use of a monoclonal antibody and a biotin-labeled polyclonal antibody produced in Example 6, an optimum antibody under the improved measuring conditions determined in Example 13 was selected. Specifically, each EDTA-plasma sample was diluted with a buffer containing EDTA and a surfactant. Subsequently, the thus-diluted specimen was added to plates which had been coated with a variety of monoclonal antibodies. After washing of the plate, a monoclonal antibody was selected through a method including addition of a biotin-labeled polyclonal antibody. The optimum monoclonal antibody selected was PPMX 0102, which was deposited as FERM AP-20395 to National Institute of Advanced Industrial Science and Technology, International Patent Organism Depositary (Central 6th, 1-1-1 Higashi, Tsukuba City, Ibaraki, 305-8566, Japan).

Example 15 Addition-recovery test of PTX3 and stability test after freezing storage of PTX3 in a plasma sample collected from healthy people, ,through conventional assay system and newly constructed assay system.
A conventional assay system (MNB4, product of ALEXIS), an assay system employing PPMX 0112, and a newly produced assay system employing PPMX 0102 (FERM AP-20395) were evaluated in terms of determination of PTX3 value added to and recovered from a plasma sample collected from healthy people. Specifically, 5-ng/mL PTX3 was added to an EDTA-plasma sample collected from a healthy volunteer. After mixing, the PTX3 level of the sample was determined. As shown in Table 6, a recovery of 86% was obtained through employment of PPMX 0112, while other assay systems exhibited PTX3 levels comparable to the amount of addition. After storage of PTX3-added plasma samples at -80°C for one day and thawing them, the newly produced assay system employing PPMX 0102 (FERM AP-20395) attained a recovery of approximately 100%, whereas the assay system employing MNB4 attained a PTX3 recovery as small as 87% (Table 7).

**[Table 6]**

| Panel specimen Added PTX3 level | PPMX 0112 (PTX3 ng/mL) | PPMX 102 (PTX3 ng/mL) | MNB4 (PTX3 ng/mL) |
|---|---|---|---|
| 0 ng/mL | 1.21 | 1.02 | 1.32 |
| 5 ng/mL | 5.52 | 6.32 | 6.66 |

**[Table 7]**

| | PPMX 0102 | | MNB4 | |
|---|---|---|---|---|
| | Frozen | Not frozen | Frozen | Not frozen |
| Antigen-added buffer measured (ng/mL) | 5.43 | 5.43 | 5.29 | 5.29 |
| Antigen-added plasma determined (ng/mL) | 6.32 | 6.31 | 5.97 | 6.66 |
| Recovery (%) | 100 | 100 | 87 | 100 |

### Example 16 Determination of blood PTX3 levels of heart disease patients

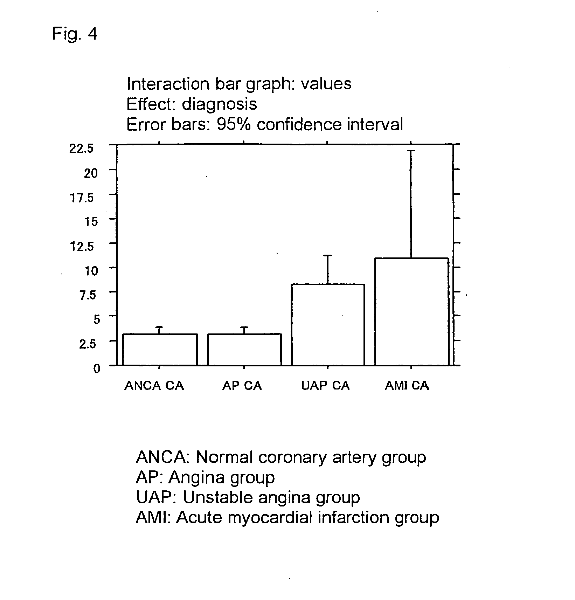

Blood samples were collected from various heart disease patients. Each sample was assayed in terms of PTX3 level through ELISA employing PPMX 0102 (FERM AP-20395), whereby a variation of PTX3 depending on pathological conditions was investigated. Specifically, the patients were divided into four groups in terms of coronary artery condition (CA): normal coronary artery (ANCA) group (25 patients); stable angina (AP) group (9 patients); unstable angina (UAP) group (3 patients); and myocardial infarction (AMI) group (3 patients). Plasma PTX3 level of each patient was determined, and obtained values were compared among the four groups. As a result, PTX3 level was considerably increased in the UAP CA group as compared with the ANCA CA group, and PTX3 level was considerably increased in the AMI CA as compared with the ANCA CA group and the AP CA group. As shown in Fig. 4, as the pathological condition becomes more severe (i.e., stable angina, unstable angina, and myocardial infarction in this order), PTX3 level clearly increases. This evidence indicates that the aggravation of a heart disease is proportionate to the aggravation of vascular injury. Thus, the determination of blood PTX3 level is expected to serve for monitoring, control of phthological conditions, and a treatment regimen of heart diseases caused by vascular injury.

### Example 17 Blood PTX3 level of cold patients

Similar to PTX3, CRP, which is an inflammation-related protein belonging to pentraxin family, is used in the diagnosis of common cold. In contrast with the CRP-producing liver cells, PTX3-producing cells are limited to vessel-forming cells such as endothelial cells and smooth muscle cells. In order to verify the vessel-specificity of PTX3, blood PTX3 levels of three cold patients were determined. As a result, as shown in Table 8, the level before the onset is 0.67 to 1.15 (average: 0.92 ng/mL) while the level after the onset is 0.79 to 1.06 (average: 0.90 ng/mL), indicating that the PTX3 levels before and after the onset are nearly identical. Therefore, PTX3 serves as a marker highly specific to the blood vessel and is not a marker for the diagnosis of systemic inflammation. Thus, PTX3 has been confirmed to be useful for assessing the extent of vascular injury.

**[Table 8]**

| | PTX3 ng/mL | | |
|---|---|---|---|
| | Sample 1 | Sample 2 | Sample 3 |
| Healthy | 0.67 | 1.15 | 0.93 |
| Cold | 0.79 | 1.06 | 0.84 |

## Claims

1. A method of assessing a grade of vascular injury, **characterized by** determining the level of PTX3 in a test sample.

2. The method according to claim 1, wherein a grade of vascular injury represents the severity of heart disease or the severity of cerebrovascular disease.

3. The method according to claim 1 or 2, wherein the test sample is blood, serum, or plasma.

4. The method according to any one of claims 1 to 3, wherein the PTX3 protein level of the test sample is determined by use of an anti-PTX3 antibody.

5. The method according to claim 4, which employs an anti-PTX3 antibody immobilized on a carrier and an anti-PTX3 antibody labeled with a labeling substance.

6. The method according to claim 5, wherein the labeling substance is biotin.

7. The method according to any one of claims 4 to 6, wherein the anti-PTX3 antibody is an antibody recognizing full-length PTX3 or a peptide fragment thereof.

8. A diagnostic agent for assessing a grade of vascular injury, the agent comprising an anti-PTX3 antibody.

9. The diagnostic agent according to claim 8, which comprises an anti-PTX3 antibody immobilized on a carrier and an anti-PTX3 antibody labeled with a labeling substance.

10. A method of screening a medicament for vascular injury, **characterized by** administering a test substance to an animal or bringing a test substance into contact with a cell, and determining variation in amount of PTX3 protein or a PTX3 gene.

11. The screening method according to claim 10, wherein the vascular injury is a heart disease or a cerebrovascular disease.
